# EUROPÄISCHE PATENTSCHRIFT

(11) **EP 0 659 727 B1**
(45) Veröffentlichungstag und Bekanntmachung des Hinweises auf die Patenterteilung: **27.08.1997**
(21) Anmeldenummer: 94119632.1
(22) Anmeldetag: 13.12.1994
(51) Int. Cl.: C07C 46/06, C07C 46/08, C07C 50/04

(54) **Verfahren zur Herstellung von Benzochinonen durch Oxidation von Phenolen**
Process for the preparation of benzoquinones by oxidation of phenoles
Procédé de préparation de benzoquinones par oxydation des phénols

(30) Priorität: 21.12.1993 DE 4343667
(43) Veröffentlichungstag der Anmeldung: 28.06.1995
(73) Patentinhaber: BASF AKTIENGESELLSCHAFT, 67056 Ludwigshafen (DE)
(72) Erfinder: Gessner, Thomas, Dr., D-69120 Heidelberg (DE)

(56) Entgegenhaltungen:
- EP-A- 0 564 930
- EP-A- 0 568 806
- DE-A- 2 138 931
- DE-A- 2 427 606
- FR-A- 2 655 985
- INORGANICA CHIM. ACTA, Bd.144, 1988 Seiten 1 - 3 K. SAKATA ET AL

## Beschreibung

Die vorliegende Erfindung betrifft ein neues Verfahren zur Herstellung von Benzochinonen durch Oxidation von Phenolen in Gegenwart eines Verdünnungsmittels und eines Sauerstoff übertragenden Katalysators, der ein komplex gebundenes Schwermetallion enthält.

Aus der DE-A-3 302 498 ist die Oxidation von 2,3,6-Trimethylphenol zu 2,3,5-Trimethyl-p-benzochinon bekannt. Als Oxidationsmittel dient Sauerstoff und als Katalysator eine Kobalt-Salcominverbindung. Gemäß der JP-A-127 937/1974 kann die gleiche Umsetzung auch mit Kobaltkomplexen von Dimethylglyoxim, Phthalocyanin oder Porphyrin vorgenommen werden. Weiterhin beschreibt die DE-A-4 029 198 diese Oxidation in Gegenwart eines Kupfer(II)halogenid-Katalysators.

In Inorganica Chim. Acta, Band 144, Seiten 1 bis 3, 1988, ist die Oxidation von verschiedenen Hydrochinonen mit Luft in Gegenwart von (Dibenzo[b,i][1,4,8,11]tetraazacyclotetradecinato)kobalt (II), -nickel (II) oder -kupfer(II) beschrieben.

Aufgabe der vorliegenden Erfindung war es, ein neues Verfahren zur Herstellung von Benzochinonen bereitzustellen, das von den entsprechenden Phenolen oder Hydrochinonen ausgeht und das die Zielprodukte auf einfache Weise in hoher Ausbeute und Reinheit liefert.

Es wurde nun gefunden, daß die Herstellung von Benzochinonen der Formel I 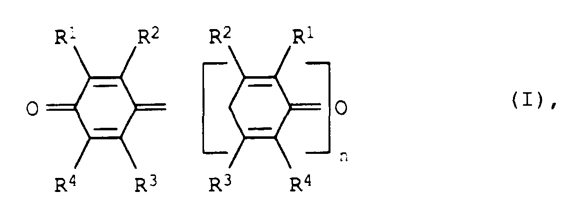 in der
R¹, R², R³ und R⁴ unabhängig voneinander jeweils Wasserstoff, C₁-C₄-Alkyl, C₁-C₄-Alkoxy, Aryloxy, C₁-C₄-Alkylsulfonyl, Arylsulfonyl, Hydroxysulfonyl, Hydroxy oder Halogen und
n 0 oder 1 bedeuten,
durch Oxidation eines Phenols der Formel II 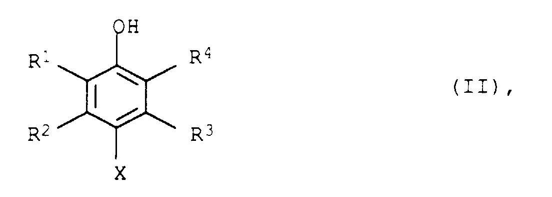 in der
X Wasserstoff oder Hydroxy bedeutet und
R¹, R², R³ und R⁴ jeweils die obengenannte Bedeutung besitzen,
in Gegenwart eines Verdünnungsmittels und eines Sauerstoff übertragenden Katalysators, der ein komplex gebundenes Schwermetallion enthält, vorteilhaft gelingt, wenn man als Oxidationsmittel Sauerstoff, Wasserstoffperoxid, eine Wasserstoffperoxid freisetzende Verbindung, ein organisches Hydroperoxid, eine Percarbonsäure oder Peroxomonoschwefelsäure oder deren Salze verwendet und wenn der Sauerstoff übertragende Katalysator aus der Klasse der Eisen-, Mangan- oder Chromtetraaza[14]annulene stammt.

Alle in den obengenannten Formeln I und II auftretenden Alkylgruppen können sowohl geradkettig als auch verzweigt sein.

Geeignete Reste R¹, R², R³ und R⁴ sind z.B. Methyl, Ethyl, Propyl, Isopropyl, Butyl, Isobutyl, sec-Butyl, tert-Butyl, Methoxy, Ethoxy, Propoxy, Isopropoxy, Butoxy, Isobutoxy, sec-Butoxy, Phenoxy, durch C₁-C₄-Alkyl, C₁-C₄-Alkoxy oder Halogen 1- bis 3-fach substituiertes Phenoxy, wie 2-, 3- oder 4-Methylphenoxy, 2-, 3- oder 4-Methoxyphenoxy, 2-, 3- oder 4-Chlorphenoxy, 2,4-Dimethylphenoxy, 2,4-Dimethoxyphenoxy oder 2,4-Dichlorphenoxy, Methylsulfonyl, Ethylsulfonyl, Propylsulfonyl, Isopropylsulfonyl, Butylsulfonyl, Phenylsulfonyl, durch C₁-C₄-Alkyl, C₁-C₄-Alkoxy oder Halogen 1- bis 3-fach substituiertes Phenylsulfonyl, Fluor, Chlor oder Brom.

Wenn die Benzochinone der Formel I Hydroxysulfonylreste aufweisen und in Salzform vorliegen, kommen als Gegenionen Metall- oder Ammoniumionen in Betracht. Metallionen sind insbesondere die Lithium-, Natrium- oder Kaliumionen. Unter Ammoniumionen im erfindungsgemäßen Sinne sind entweder unsubstituierte oder substituierte Ammoniumkationen zu verstehen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes C₁-C₂₀-Alkyl zu verstehen, das durch Hydroxylgruppen substituiert und/oder durch Sauerstoffatome in Etherfunktion unterbrochen sein kann.

Hervorzuheben sind Natrium- oder Kaliumionen.

Bevorzugt ist eine Verfahrensweise zur Herstellung von Benzochinonen der Formel I, in der n 0 bedeutet.

Weiterhin bevorzugt ist eine Verfahrensweise zur Herstellung von Benzochinonen der Formel I, in der R¹, R², R³ und R⁴ unabhängig voneinander jeweils Wasserstoff, C₁-C₄-Alkyl oder Hydroxysulfonyl bedeuten.

Besonders bevorzugt ist eine Verfahrensweise zur Herstellung von Benzochinonen der Formel I, in der R¹, R², R³ und R⁴ unabhängig voneinander jeweils Wasserstoff oder Methyl bedeuten.

Weiterhin besonders bevorzugt ist eine Verfahrensweise zur Herstellung von Benzochinonen der Formel I, in der R¹ und R³ jeweils Hydroxysulfonyl und R² und R⁴ jeweils Wasserstoff bedeuten.

Ganz besonders bevorzugt ist eine Verfahrensweise zur Herstellung von Benzochinonen der Formel I, in der R¹, R² und R³ jeweils Methyl und R⁴ Wasserstoff bedeuten.

Geeignete Katalysatoren, die Sauerstoff übertragen und ein komplex gebundenes Schwermetallion enthalten, stammen aus der Klasse der Eisen-, Mangan- oder Chromtetraaza[14]annulene. Die Schwermetallionen sind dabei in der Regel 2- oder 3-wertig.

Solche Verbindungen sind an sich bekannt und beispielsweise in der DE-A-2 427 606 beschrieben.

Sie gehorchen beispielsweise der Formel III 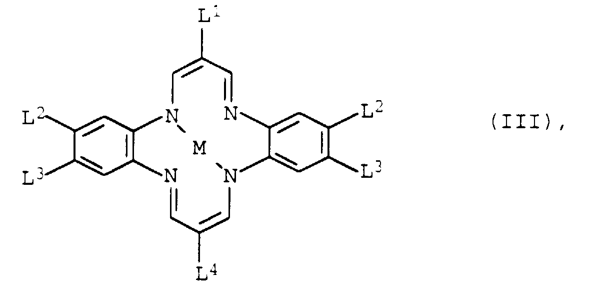 worin
- L¹: Wasserstoff, gegebenenfalls durch Hydroxysulfonyl, Carboxyl, Amino, C₁-C₄-Mono- oder Dialkylamino, Ammonium, C₁-C₄-Mono-, Di- oder Trialkylammonium oder Benzyl-C₁-C₄-dialkylammonium substituiertes C₁-C₈-Alkyl, Chlor, C₁-C₄-Alkoxy, gegebenenfalls durch Hydroxysulfonyl, Carboxyl, Amino, C₁-C₄-Mono- oder Dialkylamino, Ammonium, C₁-C₄-Mono-, Di- oder Trialkylammonium oder Benzyl-C₁-C₄-dialkylammonium substituiertes Phenyl, gegebenenfalls durch Hydroxysulfonyl, Carboxyl, Amino, C₁-C₄-Mono- oder Dialkylamino, Ammonium, C₁-C₄-Mono-, Di- oder Trialkylammonium oder Benzyl-C₁-C₄-dialkylammonium substituiertes Phenylazo, C₁-C₄-Alkoxycarbonyl, Hydroxysulfonyl oder
einen Rest der Formel 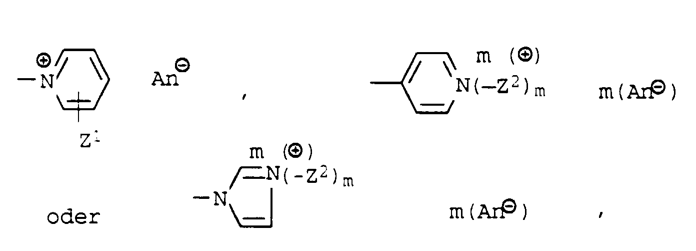 worin
m für 0 oder 1, Z¹ für Wasserstoff, C₁-C₄-Alkyl, Carboxyl, C₁-C₄-Alkoxycarbonyl oder Carbamoyl, Z² für Wasserstoff oder C₁-C₄-Alkyl und An^{⊖} für das Äquivalent eines Anions stehen,
- L² und L³: unabhängig voneinander jeweils Wasserstoff, Methyl, Hydroxysulfonyl oder L² und L³ zusammen einen anellierten Benzoring,
- L⁴: den Rest L¹ oder einen Rest der Formel 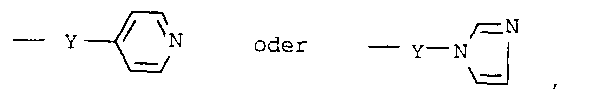 worin Y für C₁-C₈-Alkylen steht, und
- M: Eisen, Mangan oder Chrom bedeuten.

Geeignete Anionen sind z.B. Fluorid, Chlorid, Bromid, Iodid, Hydrogensulfat, Sulfat, Tetrafluoroborat, Formiat, Acetat, Propionat, Mono-, Di- oder Trichloracetat, Lactat, Methoxyacetat, Citrat, Succinat, Methylsulfonat, Benzolsulfonat oder 2- oder 4-Methylbenzolsulfonat.

Diese Anionen sind auch dann vorhanden, wenn in den Resten L¹ und/oder L⁴ durch Ammonium, Mono-, Di- oder Trialkylammonium oder Benzyldialkylammonium substituierte Reste auftreten.

Besonders bevorzugt ist die Verwendung von Eisentetraaza[14]annulenen als Katalysatoren.

Insbesondere hervorzuheben ist die Verwendung von Eisen-5,14-dihydrodibenzo[b,i][5,9,14,18]tetraaza[14]annulen als Katalysator, auch (Dibenzo[b,i][1,4,8,11]tetraazacyclotetradecinato)eisen(II) genannt.

Die Katalysatoren können homogen oder heterogen in Lösung angewandt werden. Auch ihre Fixierung auf einem Trägermaterial, z.B. Kieselgel oder Ionenaustauscherharz, ist möglich.

Als Oxidationsmittel im erfindungsgemäßen Verfahren dient Sauerstoff, Wasserstoffperoxid, eine Wasserstoffperoxid freisetzende Verbindung, ein organisches Hydroperoxid, eine Percarbonsäure oder Peroxomonoschwefelsäure oder deren Salze.

Sauerstoff kann entweder in reiner Form oder in verdünnter Form, z.B. als Luft verwendet werden. Im allgemeinen kommt er in gasförmigem Aggregatzustand zur Anwendung. Bezogen auf 1 l Reaktionsgemisch werden dabei in der Regel pro Stunde 10 bis 100 l gasförmiger Sauerstoff zugeführt.

Als Wasserstoffperoxid freisetzende Verbindungen können z.B. Alkaliperborate oder -percarbonate in Betracht kommen.

Geeignete organische Hydroperoxide sind z.B. Cumolhydroperoxid oder Alkylhydroperoxide, dabei insbesondere tert-Butylhydroperoxid.

Geeignete Percarbonsäuren sind z.B. Peressigsäure, m-Chlorperbenzoesäure, Magnesium-bis(monoperoxyphthalat)-hexahydrat oder 1,12-Dodecandipersäure.

Salze der Peroxomonoschwefelsäure sind vorzugsweise deren Alkalisalze, wie Lithium-, Natrium- oder Kaliumperoxomonosulfat. Die Verwendung von Salzen der Peroxomonoschwefelsäure, insbesondere von Natrium- oder Kaliumperoxomonosulfat ist bevorzugt. Auch handelsübliche Mischungen von Kaliumperoxomonosulfat mit Kaliumhydrogensulfat und Kaliumsulfat sind anwendbar.

Die Verwendung von Wasserstoffperoxid, tert-Butylhydroperoxid oder Magnesium-bis(monoperoxyphthalat)-hexahydrat, ist hervorzuheben.

Bei Verwendung von Wasserstoffperoxid als Oxidationsmittel wird dies in der Regel in Form einer 10 bis 70 gew.-%igen wäßrigen Lösung angewandt.

Die Verwendung der organischen Hydroperoxide geschieht im allgemeinen in Form einer ca. 70 gew.-%igen wäßrigen Lösung.

Magnesium-bis(monoperoxyphthalat)-hexahydrat kann in fester Form, als wäßrige Lösung oder als wäßrige Suspension angewandt werden.

In manchen Fällen kann es auch von Vorteil sein, das Verfahren zusätzlich in Gegenwart von geringen Mengen (im allgemeinen bis zu 2 mol-%, bezogen auf das Phenol II) einer das Oxidationsmittel stabilisierenden Verbindung, z.B. Ethylendiamintetraessigsäure, Diethylentriaminpentaessigsäure, Nitrilotriessigsäure, β-Alanindiessigsäure, Isoserindiessigsäure, Ethylendiamintetra(methylenphosphonsäure), Hexamethylendiamintetra(methylenphosphonsäure), Diethylentriaminpenta(methylenphosphonsäure) oder deren Alkalisalze, Trimethylessigsäure, p-Toluolsulfonsäure, Natriumsilicat, Aceton, Natriumfluorid, Cyanamid oder Ascorbinsäure und/oder eines Stickstoff enthaltenden aromatischen Heterocyclus, z.B. Imidazol, N-Methylimidazol, Pyridin, Pyrazol, Pyrrol oder 1,3,4-Triazol, durchzuführen.

Bei der Anwendung von Wasserstoffperoxid oder einer Wasserstoffperoxid freisetzenden Verbindung als Oxidationsmittel kann es auch von Vorteil sein, das erfindungsgemäße Verfahren zusätzlich in Gegenwart von geringen Mengen (im allgemeinen bis zu 1 Gew.-%, bezogen auf das Gewicht der Reaktionspartner) eines Entschäumers und Entlüfters durchzuführen, um den bei der Umsetzung entstehenden Sauerstoff besser abzuführen. Geeignete Verbindungen dieser Art sind z.B. langkettige Alkohole oder Phosphorsäureester.

Bei der Anwendung von Wasserstoffperoxid oder einer Wasserstoffperoxid freisetzenden Verbindung als Oxidationsmittel kann es weiterhin von Vorteil sein, das erfindungsgemäße Verfahren zusätzlich in Gegenwart von 0,1 bis 10 mol-%, vorzugsweise 1 bis 10 mol-%, jeweils bezogen auf 1 mol Phenol II, an Benzoesäure, m-Chlorbenzoesäure, phosphoriger Säure, salpetriger Säure oder eines Nitrits, vorzugsweise eines Alkalinitrits, durchzuführen. Dadurch kann sowohl die Reaktionstemperatur (20 bis 60°C, vorzugsweise 30 bis 50°C) als auch die Menge an Wasserstoffperoxid oder Wasserstoffperoxid freisetzender Verbindung verringert werden.

Weiterhin kann es von Vorteil sein, das Verfahren in Gegenwart von geringen Mengen an Schwefelsäure, in der Regel 0,1 bis 2 Gew.-% konz. Schwefelsäure, bezogen auf das Gewicht an Verdünnungsmittel, durchzuführen. Dadurch kann die Ausbeute der Benzochinone I erhöht werden.

Je mol Phenol der Formel II verwendet man in der Regel 1 bis 10 mol, vorzugsweise 1 bis 6 mol und insbesondere 1,5 bis 5 mol an Wasserstoffperoxid, 1 bis 5 mol, vorzugsweise 1 bis 3 mol und insbesondere 1,2 bis 2,5 mol organisches Hydroperoxid oder 1 bis 5 mol, vorzugsweise 1 bis 4 mol Percarbonsäure (jeweils bezogen auf einen Peroxycarboxyrest) oder 1 bis 3 mol, vorzugsweise 1,2 bis 2,5 mol Peroxomonoschwefelsäure oder deren Salze.

Der Sauerstoff übertragende Katalysator, der ein komplex gebundenes Schwermetallion enthält, wird im allgemeinen in einer Menge von 0,1 bis 5 mol-%, vorzugsweise 1 bis 3 mol-%, jeweils bezogen auf das Phenol II, angewandt.

Geeignete Verdünnungsmittel sind z.B. Wasser, Essigsäure, Chloroform, Toluol, N,N-Dimethylformamid, N-Methylpyrrolidon, Alkohole, wie Methanol, Ethanol Propanol, Isopropanol, Butanol oder Isobutanol, Glykole, wie Ethylenglykol oder Propylenglykol oder deren Gemische.

Vorzugsweise verwendet man Essigsäure oder Gemische von Wasser und Essigsäure.

Zweiphasige Gemische, die z.B. aus der Anwendung von Wasser und einem nicht oder nur begrenzt mit Wasser mischbaren Lösungsmittel resultieren, werden gegebenenfalls mit einem Phasentransferkatalysator versehen. Als Phasentransferkatalysatoren kommen dabei die üblichen, an sich bekannten Produkte, z.B. Arylsulfonate, wie Benzol- oder Toluolsulfonat, in Betracht.

Das neue Verfahren wird in der Regel bei atmosphärischem Druck und einer Temperatur von 0 bis 100°C, vorzugsweise von 20 bis 60°C durchgeführt. Es kann sowohl in kontinuierlicher als auch diskontinuierlicher Arbeitsweise vorgenommen werden.

Man führt das neue Verfahren zweckmäßig so durch, daß man zunächst den Katalysator, vorzugsweise gelöst im Verdünnungsmittel, und gegebenenfalls die Hilfsmittel vorlegt und dann, vorzugsweise gleichzeitig, eine Lösung des Phenols II im Verdünnungsmittel, gegebenenfalls in Gegenwart einer Säure, und das Oxidationsmittel unter Rühren zuführt.

Es ist aber auch möglich, Katalysator und Phenol II zusammen mit dem Verdünnungsmittel vorzulegen und dann das Oxidationsmittel unter Rühren zuzuführen.

In der Regel wählt man dabei ein Gewichtsverhältnis Phenol II: Verdünnungsmittel von 1:3 bis 1:40, vorzugsweise von 1:3 bis 1:10.

Die Oxidation erfolgt unter Rühren bei der obengenannten Temperatur. Nach Zugabe des Oxidationsmittels ist die Umsetzung im allgemeinen beendet. Das Zielprodukt kann dann auf üblichem Weg, z.B. mittels Wasserdampfdestillation oder durch Abfiltrieren, isoliert werden.

Das erfindungsgemäße Verfahren ist einfach durchzuführen und liefert die Benzochinone der Formel I in guter Ausbeute und hoher Reinheit.

Bei den Benzochinonen der Formel I handelt es sich um wertvolle Zwischenprodukte für die Herstellung von Wirkstoffen, z.B. von α-Tocopherol (Vitamin E).

Die folgenden Beispiele sollen die Erfindung näher erläutern.

### Beispiel 1

Zu einer Lösung von 0,68 g (2,0 mmol) Eisen-5,14-dihydrodibenzo[b,i][5,9,14,18]tetraaza[14]annulen, 2,52 g (2,0 mmol) einer 40 gew.-%igen wäßrigen Lösung des Pentanatriumsalzes der Diethylentriaminpentaessigsäure und 0,5 ml konz. Schwefelsäure in 50 ml Essigsäure wurden gleichzeitig eine Lösung von 13,6 g (100 mmol) 2,3,6-Trimethylphenol in 40 ml Essigsäure und 62,3 g (550 mmol) 30 gew.-%iges wäßriges Wasserstoffperoxid bei 40°C unter Rühren und Kühlen zugetropft. Anschließend wurde der Reaktionsansatz einer Wasserdampfdestillation unterworfen. Das Destillat wurde mit Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und zu einem gelben Öl eingeengt, das langsam erstarrte. Man enthielt 12,6 g (84 %) 2,3,5-Trimethyl-p-benzochinon in Form von gelben öligen Kristallen.

### Beispiel 2

Zu einer Lösung von 0,68 g (2,0 mmol) Eisen-5,14-dihydrodibenzo[b,i][5,9,14,18]tetraaza[14]annulen und 0,5 ml konz. Schwefelsäure in 50 ml Essigsäure wurden gleichzeitig eine Lösung von 13,6 g (100 mmol) 2,3,6-Trimethylphenol in 40 ml Essigsäure und eine Lösung von 32,2 g (250 mmol) 70 gew.-%igem wäßrigem tert-Butylhydroperoxid in 20 ml Essigsäure bei 40°C unter Rühren und Kühlen zugetropft. Anschließend wurde der Reaktionsansatz einer Wasserdampfdestillation unterworfen. Das Destillat wurde mit Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und zu einem gelben Öl eingeengt, das langsam erstarrte. Man enthielt 14,0 g (93 %) 2,3,5-Trimethyl-p-benzochinon in Form von gelben öligen Kristallen.

### Beispiel 3

Zu einer Lösung von 0,68 g (2,0 mmol) Eisen-5,14-dihydrodibenzo[b,i][5,9,14,18]tetraaza[14]annulen und 0,5 ml konz. Schwefelsäure in 50 ml Essigsäure wurden gleichzeitig eine Lösung von 13,6 g (100 mmol) 2,3,6-Trimethylphenol in 40 ml Essigsäure und eine Lösung von 73,7 g (240 mmol) 50 gew.-%igem Kaliumperoxomonosulfat in 320 ml Wasser bei Raumtemperatur unter Rühren und Kühlen zugetropft. Anschließend wurde der Reaktionsansatz einer Wasserdampfdestillation unterworfen. Das Destillat wurde mit Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und zu einem gelben Öl eingeengt, das langsam erstarrte. Man enthielt 11,1 g (74 %) 2,3,5-Trimethyl-p-benzochinon in Form von gelben öligen Kristallen.

### Beispiel 4

Zu einer Lösung von 0,68 g (2,0 mmol) Eisen-5,14-dihydrodibenzo[b,i][5,9,14,18]tetraaza[14]annulen, 2,52 g (2,0 mmol) einer 40 gew.-%igen wäßrigen Lösung des Pentanatriumsalzes der Diethylentriaminpentaessigsäure und 0,5 ml konz. Schwefelsäure in 50 ml Essigsäure wurden gleichzeitig eine Lösung von 15,2 g (100 mmol) 2,3,5-Trimethylhydrochinon in 150 ml Essigsäure und 28,4 g (250 mmol) 30 gew.-%iges wäßriges Wasserstoffperoxid bei 40°C unter Rühren und Kühlen zugetropft. Anschließend wurde der Reaktionsansatz einer Wasserdampfdestillation unterworfen. Das Destillat wurde mit Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und zu einem gelben Öl eingeengt, das langsam erstarrte. Man enthielt 12,9 g (86 %) 2,3,5-Trimethyl-p-benzochinon in Form von gelben öligen Kristallen.

### Beispiel 5

Zu einer Lösung von 0,68 g (2,0 mmol) Eisen-5,14-dihydrodibenzo[b,i][5,9,14,18]tetraaza[14]annulen und 0,5 ml konz. Schwefelsäure in 50 ml Essigsäure wurden gleichzeitig eine Lösung von 15,2 g (100 mmol) 2,3,5-Trimethylhydrochinon in 150 ml Essigsäure und 16,1 g (125 mmol) 70 gew.-%iges wäßriges tert-Butylhydroperoxid bei 40°C unter Rühren und Kühlen zugetropft. Anschließend wurde der Reaktionsansatz einer Wasserdampfdestillation unterworfen. Das Destillat wurde mit Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und zu einem gelben Öl eingeengt, das langsam erstarrte. Man enthielt 11,7 g (78 %) 2,3,5-Trimethyl-p-benzochinon in Form von gelben öligen Kristallen.

### Beispiel 6

Zu einer Lösung von 12,2 g (50 mmol) 2,6-Dimethylphenol, 0,34 g (1,0 mmol) Eisen-5,14-dihydrodibenzo[b,i][5,9,14,18[tetraaza[14]annulen, 1,26 g (1,0 mmol) einer 40 gew.-%igen wäßrigen Lösung des Pentanatriumsalzes der Diethylentriaminpentaessigsäure und 0,5 ml konz. Schwefelsäure in 50 ml Essigsäure wurden 28,4 g (250 mmol) 30 gew.-%iges wäßriges Wasserstoffperoxid bei 40°C unter Rühren und Kühlen zugetropft. Nach dem Abkühlen auf Raumtemperatur wurde der Feststoff abfiltriert und aus Essigsäure umkristallisiert. Man erhielt 7,4 g (62 %) 3,3',5,5'-Tetramethyldiphenochinon in Form von dunkelroten Nadeln. Schmp.: 207 bis 225°C (Zers.).

### Beispiel 7

Zu einer Lösung von 0,68 g (2,0 mmol) Eisen-5,14-dihydrodibenzo[b,i][5,9,14,18]tetraaza[14]annulen, 2,52 g (2,0 mmol) einer 40 gew.-%igen wäßrigen Lösung des Pentanatriumsalzes der Diethylentriaminpentaessigsäure und 0,5 ml konz. Schwefelsäure in 50 ml Essigsäure wurden gleichzeitig eine Lösung von 11,0 g (100 mmol) Hydrochinon in 250 ml Essigsäure und 28,4 g (250 mmol) 30 gew.-%iges wäßriges Wasserstoffperoxid bei 40°C unter Rühren und Kühlen zugetropft. Es wurde 15 min nachgerührt. Anschließend wurde der Reaktionsansatz einer Wasserdampfdestillation unterworfen. Das Destillat wurde dreimal mit insgesamt 750 ml Methylenchlorid ausgeschüttelt. Die organische Phase wurde mit waßriger Natriumhydrogencarbonatlösung und dann mit Wasser geschüttelt, von der wäßrigen Phase abgetrennt, über Magnesiumsulfat getrocknet, filtriert und bis zur Trockne eingeengt. Man enthielt 6,8 g (63 %) Benzochinon in Form eines gelben Pulvers. Schmp.: 113 bis 115°C.

### Beispiel 8

Zu einer Lösung von 0,68 g (2,0 mmol) Eisen-5,14-dihydrodibenzo[b,i][5,9,14,18]tetraaza[14]annulen, 2,52 g (2,0 mmol) einer 40 gew.-%igen wäßrigen Lösung des Pentanatriumsalzes der Diethylentriaminpentaessigsäure und 0,5 ml konz. Schwefelsäure in 50 ml Essigsäure wurde gleichzeitig eine Lösung von 12,4 g (100 mmol) Methylhydrochinon in 90 ml Essigsäure und 28,4 g (250 mmol) 30 gew.-%iges wäßriges Wasserstoffperoxid bei 40°C unter Rühren und Kühlen zugetropft. Es wurde 15 min nachgerührt. Anschließend wurde der Reaktionsansatz einer Wasserdampfdestillation unterworfen. Das Destillat wurde dreimal mit insgesamt 400 ml Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und bis zur Trockne eingeengt. Man enthielt 10,9 g (89 %) Methylbenzochinon in Form eines gelben Pulvers. Schmp.: 69 bis 71°C.

### Beispiel 9

Zu einer Lösung von 0,68 g (2,0 mmol) Eisen-5,14-dihydrodibenzo[b,i][5,9,14,18]tetraaza[14]annulen, 2,52 g (2,0 mmol) einer 40 gew.-%igen wäßrigen Lösung des Pentanatriumsalzes der Diethylentriaminpentaessigsäure und 0,5 ml konz. Schwefelsäure in 50 ml Essigsäure wurde gleichzeitig eine Lösung von 13,8 g (100 mmol) 2,3-Dimethylhydrochinon in 500 ml Essigsäure und 28,4 g (250 mmol) 30 gew.-%iges wäßriges Wasserstoffperoxid bei 40°C unter Rühren und Kühlen zugetropft. Es wurde 15 min nachgerührt. Anschließend wurde der Reaktionsansatz einer Wasserdampfdestillation unterworfen. Das Destillat wurde dreimal mit insgesamt 400 ml Methylenchlorid ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und bis zur Trockne eingeengt. Man enthielt 11,8 g (87 %) 2,3-Dimethylbenzochinon in Form eines gelben Pulvers. Schmp.: 57 bis 59°C.

### Beispiel 10

34,6 g (100 mmol) 2,5-Dihydroxybenzol-1,4-disulfonsäure-Dikaliumsalz wurden in 500 ml Wasser bei 60°C gelöst. Nach der Zugabe von 0,68 g (2 mmol) Eisen-5,14-dihydrodibenzo[b,i][5,9,14,18]tetraaza[14]annulen, 0,8 g (2 mmol) Diethylentriaminpentaessigsäure, 1 ml konz. Schwefelsäure und 1 ml eines handelsüblichen Entschäumers wurden bei 60°C 28,4 g (250 mmol) 30 gew.%iges wäßriges Wasserstoffperoxid zugetropft. Es wurde 15 min bei 60°C nachgerührt. Nach dem Abkühlen der Lösung auf 5 bis 10°C wurde der Feststoff abgesaugt und aus Wasser umkristallisiert. Man erhielt 14,5 g (42 %) 1,4-Benzochinon-2,5-disulfonsäure-Dikaliumsalz in Form von rotbraune Nadeln. Schmp.: > 360°C.

### Beispiel 11

Eine Lösung von 0,68 g (2 mmol) Eisen-5,14-dihydrodibenzo[b,i][5,9,14,18]tetraaza[14]annulen in 150 ml 85 gew-%iger wäßriger Essigsäure wurde mit Sauerstoff begast. Während der Begasung wurde eine Lösung von 13,6 g (100 mmol) 2,3,6-Trimethylphenol und 0,5 g konz. Schwefelsäure in 75 ml 85 gew.-%iger Essigsäure unter kräftigem Rühren bei 40°C zugetropft. Nachdem kein Sauerstoff mehr aufgenommen wurde, wurde der Reaktionsansatz einer Wasserdampfdestillation unterworfen. Das Destillat wurde mit tert-Butylmethylether ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und bis zur Trockene eingeengt. Man erhielt 12,0 g (80%) 2,3,5-Trimethylbenzochinon in Form von gelben öligen Kristallen.

### Beispiel 12

Eine Lösung von 0,68 g (2 mmol) Eisen-5,14-dihydrodibenzo[b,i][5,9,14,18]tetraaza[14]annulen in 150 ml 85 gew-%iger wäßriger Essigsäure wurde mit Sauerstoff begast. Während der Begasung wurde eine Lösung von 11,0 g (100 mmol) Hydrochinon und 0,5 g konz. Schwefelsäure in 75 ml 85 gew.-%iger Essigsäure unter kräftigem Rühren bei 40°C zugetropft. Nachdem kein Sauerstoff mehr aufgenommen worden war, wurde der Reaktionsansatz einer Wasserdampfdestillation unterworfen. Das Destillat wurde mit tert-Butylmethylether ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und bis zur Trockene eingeengt. Man erhielt 5,0 g (46%) Benzochinon in Form eines gelben Pulvers. Schmp.: 109 bis 110°C.

### Beispiel 13

Eine Lösung von 0,68 g (2 mmol) Eisen-5,14-dihydrodibenzo[b,i][5,9,14,18]tetraaza[14]annulen in 150 ml 85 gew-%iger wäßriger Essigsäure wurde mit Sauerstoff begast. Während der Begasung wurde eine Lösung von 12,4 g (100 mmol) Methylhydrochinon und 0,5 g konz. Schwefelsäure in 75 ml 85 gew.-%iger Essigsäure unter kräftigem Rühren bei 40°C zugetropft. Nachdem kein Sauerstoff mehr aufgenommen worden war, wurde der Reaktionsansatz einer Wasserdampfdestillation unterworfen. Das Destillat wurde mit tert-Butylmethylether ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und bis zur Trockene eingeengt. Man erhielt 7,5 g (46%) 2-Methylbenzochinon in Form eines gelben Pulvers. Schmp.: 66 bis 68°C.

### Beispiel 14

Eine Lösung von 0,68 g (2 mmol) Eisen-5,14-dihydrodibenzo[b,i)[5,9,14,18]tetraaza[14]annulen und 0,5 g konz. Schwefelsäure in 150 ml 85 gew-%iger wäßriger Essigsäure wurde mit Sauerstoff begast. Während der Begasung wurde eine Lösung von 12,4 g (100 mmol) 2,3-Dimethylhydrochinon in 75 ml 85 gew.-%iger Essigsäure unter kräftigem Rühren bei 40°C zugetropft. Nachdem kein Sauerstoff mehr aufgenommen worden war, wurde der Reaktionsansatz einer Wasserdampfdestillation unterworfen. Das Destillat wurde mit tert-Butylmethylether ausgeschüttelt. Die organische Phase wurde mit wäßriger Natriumhydrogencarbonatlösung und dann mit Wasser geschüttelt, von der wäßrigen Phase abgetrennt, über Magnesiumsulfat getrocknet, filtriert und bis zur Trockene eingeengt. Man erhielt 13,0 g (95%) 2,3-Dimethylbenzochinon in Form eines gelben Pulvers. Schmp.: 56 bis 57°C.

## Patentansprüche

1. Verfahren zur Herstellung von Benzochinonen der Formel I 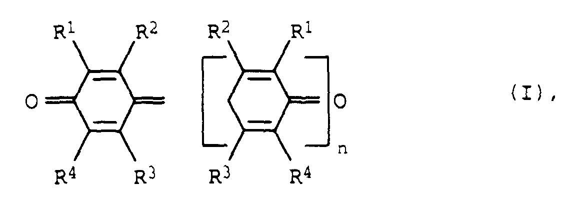 in der
R¹, R², R³ und R⁴ unabhängig voneinander jeweils Wasserstoff, C₁-C₄-Alkyl, C₁-C₄-Alkoxy, Aryloxy, C₁-C₄-Alkylsulfonyl, Arylsulfonyl, Hydroxysulfonyl, Hydroxy oder Halogen und
n 0 oder 1 bedeuten,
durch Oxidation eines Phenols der Formel II 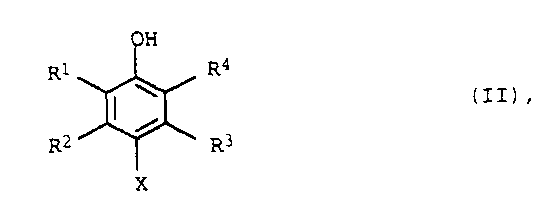 in der
X Wasserstoff oder Hydroxy bedeutet und
R¹, R², R³ und R⁴ jeweils die obengenannte Bedeutung besitzen,
in Gegenwart eines Verdünnungsmittels und eines Sauerstoff übertragenden Katalysators, der ein komplex gebundenes Schwermetallion enthält, dadurch gekennzeichnet, daß man als Oxidationsmittel Sauerstoff, Wasserstoffperoxid, eine Wasserstoffperoxid freisetzende Verbindung, ein organisches Hydroperoxid, eine Percarbonsäure oder Peroxomonoschwefelsäure oder deren Salze verwendet und daß der Sauerstoff übertragende Katalysator aus der Klasse der Eisen-, Mangan- oder Chromtetraaza[14]annulene stammt.

2. Verfahren nach Anspruch 1, dadurch gekennzeichnet, daß n 0 bedeutet.

3. Verfahren nach Anspruch 1, dadurch gekennzeichnet, daß R¹, R², R³ und R⁴ unabhängig voneinander jeweils Wasserstoff, C₁-C₄-Alkyl oder Hydroxysulfonyl bedeuten.

4. Verfahren nach Anspruch 1, dadurch gekennzeichnet, daß R¹, R², R³ und R⁴ unabhängig voneinander jeweils Wasserstoff oder Methyl bedeuten.

5. Verfahren nach Anspruch 1, dadurch gekennzeichnet, daß R¹, R² und R³ jeweils Methyl und R⁴ Wasserstoff bedeuten.

6. Verfahren nach Anspruch 1, dadurch gekennzeichnet, daß der Sauerstoff übertragende Katalysator aus der Klasse der Eisentetraaza[14]annulene stammt.

## Claims

1. A process for preparing benzoquinones of the formula I 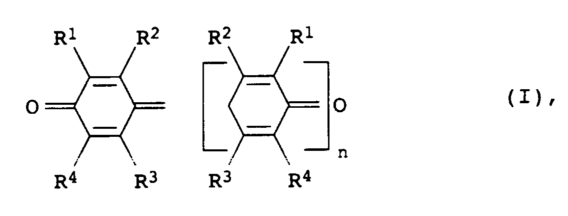 where R¹, R², R³ and R⁴ are, independently of one another, each hydrogen, C₁-C₄-alkyl, C₁-C₄-alkoxy, aryloxy, C₁-C₄-alkylsulfonyl, arylsulfonyl, hydroxysulfonyl, hydroxyl or halogen, and
n is 0 or 1,
by oxidizing a phenol of the formula II 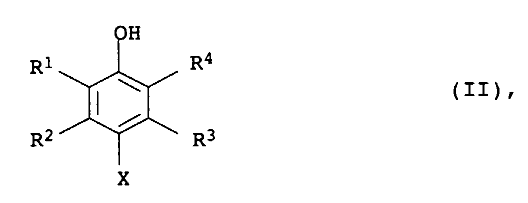 where X is hydrogen or hydroxyl, and
R¹, R², R³ and R⁴ each have the abovementioned meanings, in the presence of a diluent and of an oxygen-transferring catalyst which contains a heavy metal ion bound in a complex, wherein oxygen, hydrogen peroxide, a compound which liberates hydrogen peroxide, an organic hydroperoxide, a percarboxylic acid or peroxomonosulfuric acid or salts thereof are used as oxidizing agent, and wherein the oxygen-transferring catalyst is from the class of iron, manganese or chromium tetraaza[14]annulenes.

2. A process as claimed in claim 1, wherein n is 0.

3. A process as claimed in claim 1, wherein R¹, R², R³ and R⁴ are, independently of one another, each hydrogen, C₁-C₄-alkyl or hydroxysulfonyl.

4. A process as claimed in claim 1, wherein R¹, R², R³ and R⁴ are, independently of one another, each hydrogen or methyl.

5. A process as claimed in claim 1, wherein R¹, R² and R³ are each methyl and R⁴ is hydrogen.

6. A process as claimed in claim 1, wherein the oxygen-transferring catalyst is from the class of iron tetraaza[14]annulenes.

## Revendications

1. Procédé de fabrication de benzoquinones de formule I 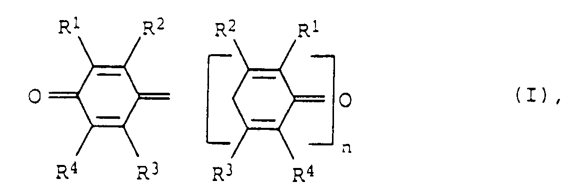 dans laquelle
R¹, R², R³ et R⁴ représentent chacun indépendamment les uns des autres un atome d'hydrogène, un groupement alkyle en C₁-C₄, alcoxy en C₁-C₄, aryloxy, alkylsulfonyle en C₁-C₄, arylsulfonyle, hydroxysulfonyle, hydroxy ou un atome d'halogène et
n vaut 0 ou 1
par oxydation d'un phénol de formule II 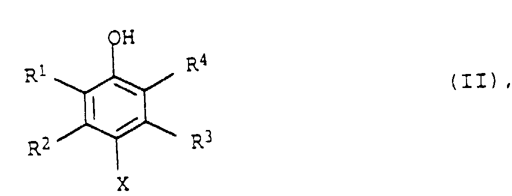 dans laquelle
X représente un atome d'hydrogène ou un groupement hydroxy et R¹, R², R³ et R⁴ prennent chacun la signification mentionnée plus haut, en présence d'un diluant et d'un catalyseur de transfert d'oxygène, qui contient un ion de métal lourd complexé, caractérisé en ce que l'on utilise comme oxydant, de l'oxygène, du peroxyde d'hydrogène, un composé libérant du peroxyde d'hydrogène, un hydroperoxyde organique, un peracide carboxylique ou de l'acide peroxomonosulfurique ou leurs sels, et en ce que le catalyseur de transfert d'oxygène est choisi dans la classe des tétraaza[14]annulènes de fer, de manganèse ou de chrome.

2. Procédé selon la revendication 1, caractérisé en ce que n vaut 0.

3. Procédé selon la revendication 1, caractérisé en ce que R¹, R², R³ et R⁴ représentent chacun indépendamment les uns des autres un atome d'hydrogène, un groupement alkyle en C₁-C₄ ou hydroxysulfonyle.

4. Procédé selon la revendication 1, caractérisé en ce que R¹, R², R³ et R⁴ représentent chacun indépendamment les uns des autres un atome d'hydrogène ou un groupement méthyle.

5. Procédé selon la revendication 1, caractérisé en ce que R¹, R² et R³ représentent chacun un groupement méthyle et R⁴ un atome d'hydrogène.

6. Procédé selon la revendication 1, caractérisé en ce que le catalyseur de transfert d'oxygène est choisi dans la classe des tétraaza[14]annulènes de fer.
